Europäisches Patentamt

European Patent Office

Office européen des brevets

(11) Veröffentlichungsnummer: **0 361 077 A2**

## (12) EUROPÄISCHE PATENTANMELDUNG

(21) Anmeldenummer: 89115506.1

(22) Anmeldetag: 23.08.89

(51) Int. Cl.5: **A61K 31/00 , A61K 31/55 , A61K 37/02**

(30) Priorität: 24.08.88 DE 3828678

(43) Veröffentlichungstag der Anmeldung:
04.04.90 Patentblatt 90/14

(84) Benannte Vertragsstaaten:
**AT BE CH DE ES FR GB GR IT LI LU NL SE**

(71) Anmelder: **BOEHRINGER INGELHEIM KG**

**D-6507 Ingelheim am Rhein(DE)**
(84) **BE CH DE ES FR GR IT LI LU NL SE AT**

Anmelder: **BOEHRINGER INGELHEIM INTERNATIONAL G.M.B.H.**

**D-6507 Ingelheim am Rhein(DE)**
(84) **GB**

(72) Erfinder: **Lohmann, Helmut, Dr.**
**Ludwig-Richter-Strasse 1**
**D-6507 Ingelheim am Rhein(DE)**
Erfinder: **Meade, Christopher John Montague, Dr.**
**Burgstrasse 104**
**D-6530 Bingen-Büdesheim(DE)**
Erfinder: **Frölke, Wilhelm, Dr.-Dipl.-Chem.**
**Veit-Stoss-Strasse 7**
**D-6507 Ingelheim am Rhein(DE)**

(54) **Verwendung von PAF-Antagonisten zur Behandlung von Autoimmunerkrankungen.**

(57) Die Erfindung betrifft die Verwendung von PAF-Antagonisten zur Behandlung von Autoimmunerkrankungen, allein oder in Kombination mit einer immunsuppressiv wirkenden Substanz z.B. Ciclosporin A. Als PAF-Antagonisten kommen insbesondere Thienotriazolodiazepine oder Triazolobenzodiazepine in Betracht. Es wird die Wirkung dieser Substanzen bei idiopathischer thrombocytopenischer Purpura (als Autoimmunerkrankung) beschrieben.

EP 0 361 077 A2

## Verwendung von PAF-Antagonisten zur Behandlung von Autoimmunerkrankungen

Die Erfindung betrifft die Verwendung von PAF-Antagonisten zur Behandlung von Autoimmunerkrankungen.

Bei der idiopathischen thrombozytopenischen Purpura (ITP) kommt es durch einen fehlgeleiteten Immunmechanismus zu einer massiven Zerstörung der eigenen Thrombozyten, die letztlich in der Milz eliminiert werden. Die Thrombozytenüberlebenszeit beträgt statt 10 Tagen oft nur wenige Stunden und die Megakariozyten im Knochenmark als Bildner der Thrombozyten sind kompensatorisch um ein Vielfaches vermehrt.

Früher war diese Erkrankung in 60-80% der Fälle tödlich. Seit der Therapie mit Steroiden, insbesondere mit Prednisolon, hat sich die Prognose der ITP deutlich verbessert.

Bei dem vorliegenden Krankheitsbild ist es erforderlich, daß die Therapie, d.h. die medikamentöse Behandlung in zahlreichen Fällen zeitlebens aufrechterhalten werden muß. Die Nachteile einer langandauernden Cortisontherapie sind dem Fachmann hinreichend bekannt.

Überraschenderweise hat sich gezeigt, daß PAF-Antagonisten (PAF-Acether-Antagonisten) erfolgreich in der Therapie zur Behandlung von Autoimmunerkrankung, insbesondere der idiopathischen thrombozytopenischen Purpura, eingesetzt werden können, ohne daß hierbei die bekannten Nebenwirkungen einer Steroidtherapie auftreten.

Verbindungen, die als PAF-Antagonisten (PAF = platelet ectivating fator) Verwendung finden, sind in der Patentliteratur und der fachspezifischen Literatur bekannt, so z.B. in Prostaglandins 35, 781 (1988). Bekannte PAF-Antagonisten sind beispielsweise die Tetrahydrofuranderivate SDZ 63-441, $\overline{S}$DZ 63-675, L 659.886. BN 52 111, Thiazolderivate des Typs RP 59 227, Imidazo[2,1-a]isochinoline des Typs SDZ 64412 und SDZ 63-135, Dihydropyridine Verbindungen des Typs SRI 63441, RO 19-3704, CV 6209 sowie Verbindungen des Hetrazepintyps wie z.B. Thienodiazepine und Benzodiazepine, wie sie in den europäischen Patentanmeldungen 176 928, 176 927, 176 929, 268 242, 315 698, 316 456, 320 992 beansprucht sind. Bevorzugt sind PAF-Antagonisten der europäischen Patentanmeldungen 230 942, 240 899, 194 416, 254 245, 268 242 und 255 028, auf die hiermit inhaltlich Bezug genommen wird. Besonders bevorzugt sind die nachfolgend genannten Verbindungen.

[4-(2-Chlorphenyl)-9-methyl-6H-thieno[3,2-f][1,2,4]triazolo-[4,3-a][1,4]diazepin-2-yl]-carbonsäuremorpholid.

[4-(2-Chlorphenyl)-9-methyl-6H-thieno[3,2-f][1,2,4]triazolo-[4,3-a][1,4]diazepin-2-yl]-carbonsäureamid

2-[4-(2-Chlorphenyl)-9-methyl-6H-thieno[3,2-f][1,2,4]triazolo[4,3-a][1,4]diazepin-2-yl]-ethan-1-carbonsäurediethylamid

2-[4-(2-Chlorphenyl)-9-methyl-6H-thieno[3,2-f][1,2,4]triazolo[4,3-a][1,4]diazepin-2-yl]-ethan-1-carbonsäure-N,N-di-(2-hydroxyethyl)amid]

2-[4-(2-Chlorphenyl)-9-methyl-6H-thieno[3,2-f][1,2,4]triazolo[4,3-a][1,4]diazepin-2-yl]-ethan-1-carbonsäuremethylamid

2-[4-(2-Chlorphenyl)-9-methyl-6H-thieno[3,2-f][1,2,4]triazolo[4,3-a][1,4]diazepin-2-yl]-ethan-1-carbonsäureisopropylamid

2-[4-(2-Chlorphenyl)-9-methyl-6H-thieno[3,2-f][1,2,4]triazolo[4,3-a][1,4]diazepin-2-yl]-ethan-1-carbonsäuredimethylamid

2-[4-(2-Chlorphenyl)-9-methyl-6H-thieno[3,2-f][1,2,4]triazolo[4,3-a][1,4]diazepin-2-yl]-ethan-1-carbonsäure-N′-methyl-piperazid)

2-[4-(2-Chlorphenyl)-9-methyl-6H-thieno[3,2-f][1,2,4]triazolo[4,3-a][1,4]diazepin-2-yl]-ethan-1-carbonsäurepyrrolidid

2-[4-(2-Chlorphenyl)-9-methyl-6H-thieno[3,2-f][1,2,4]triazolo[4,3-a][1,4]diazepin-2-yl]-ethan-1-carbonsäurepiperidid

2-[4-(2-Chlorphenyl)-6H-thieno[3,2-f][1,2,4]triazolo[4,3-a][1,4]diazepin-2-yl]-ethan-1-carbonsäuremorpholid

2-[4-(2-Chlorphenyl)-9-brom-6H-thieno[3,2-f][1,2,4]triazolo[4,3-a][1,4]diazepin-2-yl]-ethan-1-carbonsäuremorpholid

2-[4-(2-Chlorphenyl)-9-methoxy-6H-thieno[3,2-f][1,2,4]triazolo[4,3-a][1,4]diazepin-2-yl]-ethan-1-carbonsäuremorpholid

4-(Morpholin-4-yl-carbonyl)-6-(2-chlorphenyl)-11-methyl-2,3,4,5-tetrahydro-8H-[1]benzothieno[3,2-f][1,2,4]-triazolo[4,3-a][1,4]diazepin

3-(Morpholin-4-yl-carbonyl)-6-(2-chlorphenyl)-11-methyl-2,3,4,5-tetrahydro-8H-[1]benzothieno[3,2-f][1,2,4]-triazolo[4,3-a][1,4]diazepin

3-(N,N-Diethylaminocarbonyl)-6-(2-chlorphenyl)-11-methyl-2,3,4,5-tetrahydro-8H-[1]benzothieno[3,2-f][1,2,4]-triazolo[4,3-a][1,4]diazepin

4-(N,N-Diethylaminocarbonyl)-6-(2-chlorphenyl)-11-methyl-2,3,4,5-tetrahydro-8H-[1]benzothieno[3,2-f][1,2,4]-triazolo[4,3-a][1,4]diazepin

4-(4-Methylpiperazinylcarbonyl)-6-(2-chlorphenyl)-11-methyl-2,3,4,5-tetrahydro-8H-[1]benzothieno[3,2-f]-[1,2,4]triazolo[4,3-a][1,4]diazepin

4-(N-Methyl-N-2-hydroxyethylaminocarbonyl)-6-(2-chlorphenyl)-11-methyl-2,3,4,5-tetrahydro-8H-[1]-benzothieno[3,2-f][1,2,4]-triazolo[4,3-a][1,4]diazepin

4-(N,N-Dimethylaminocarbonyl)-5-(2-chlorphenyl)-10-methyl-3,4-dihydro-2H,7H-cyclopenta[4,5]thieno[3,2-f]-[1,2,4] triazolo[4,3-a][1,4]diazepin

3-(N,N-Diethylaminocarbonyl-5-(2-chlorphenyl)-10-methyl-3,4-dihydro-2H,7H-cyclopenta[4,5]thieno[3,2-f]-[1,2,4]triazolo[4,3-a] [1,4]diazepin

3-(N,N-Diethylaminocarbonyl-5-(2-chlorphenyl)-10-brom-3,4-dihydro-2H,7H-cyclopenta[4,5]thieno(3,2-f]-[1,2,4]triazolo[4,3-a] [1,4]diazepin

4-(Morpholin-4-yl-carbonylmethylaminocarbonyl-5-(2-chlorphenyl)-10-brom-3,4-dihydro-2H,7H-cyclopenta-[4,5]thieno[3,2-f]-[1,2,4]triazolo[4,3-a][1,4]diazepin

4-(Morpholin-4-yl-carbonyl)-6-(2-chlorphenyl)-11-methyl-2,3,4,5-tetrahydro-8H-[1]benzothieno[3,2-f]imidazo-[1,2-a][1,4]diazepin

4-(N,N-Diethylamino)-6-(2-chlorphenyl)-11-methyl-2,3,4,5-tetrahydro-8H-[1]benzothieno[3,2-f]imidazo[1,2-a]-[1,4]diazepin

3-(N-Morpholinomethyl)-5-(2-chlorphenyl)-10-methyl-3,4-dihydro-2H,7H-cyclopenta[4,5]thieno[3,2-f][1,2,4]-triazolo[4,3-a][1,4]diazepin

3-(Acetoxymethyl)-5-(2-chlorphenyl)-10-methyl-3,4-dihydro-2H,7H-cyclopenta[4,5]thieno[3,2-f][1,2,4]triazolo-[4,3-a][1,4]diazepin

3-(1.2.4-Triazolyl-1-yl-methyl)-5-(2-chlorphenyl)-10-methyl-3,4-dihydro-2H,7H-cyclopenta[4,5]thieno[3,2-f]-[1,2,4]triazolo[4,3-a][1,4]diazepin

3-(N-2.6-Dimethylmorpholino-methyl)-5-(2-chlorphenyl)-10-methyl-3,4-dihydro-2H,7H-cyclopenta[4,5]thieno-[3,2-f][1,2,4]triazolo-[4,3-a][1,4]diazepin

3-Acetoxymethyl-5-(2-chlorphenyl)-3,4-dihydro-2H,7H-cyclopenta[4,5]-thieno[3,2-f][1,2,4]triazolo[4,3-a][1,4]-diazepin

4-Acetoxymethyl-6-(2-chlorphenyl)-11-methyl-2,3,4,5-tetrahydro-8H-[1]benzothieno[3,2-f]imidazo[1,2-a][1,4]-diazepin

3-Acetoxymethyl-5-(2-chlorphenyl)-10-methyl-3,4-dihydro-2H,7H-cyclopenta[4,5]thieno[3,2-f]imidazo[1,2-a]-[1,4]diazepin

3-Diethylaminomethyl-5-(2-chlorphenyl)-10-methyl-3,4-dihydro-2H,7H-cyclopenta[4,5]thieno[3,2-f][1,2,4]-triazolo[4,3-a][1,4]diazepin

4-Hydroxymethyl-6-(2-chlorphenyl)-11-methyl-2,3,4,5-tetrahydro-8H-[1]benzothieno[3,2-f][1,2,4]triazolo[4,3-a][1,4]diazepin

4-(N-Morpholinomethyl)-6-(2-chlorphenyl)-11-methyl-2,3,4,5-tetrahydro-8H-[1]benzothieno[3,2-f][1,2,4]-triazolo[4,3-a][1,4]diazepin

4-(Pyrazolyl-1-yl-methyl)-6-(2-chlorphenyl)-2,3,4,5-tetrahydro-8H-[1]benzothieno[3,2-f][1,2,4]triazolo[4,3-a]-[1,4]diazepin

4-[4,4-Dimethyl-2-oxazolin-2-yl]-6-(2-chlorphenyl)-11-methyl-2,3,4,5-tetrahydro-8H-[1]benzothieno[3,2-f]-[1,2,4]triazolo[4,3-a][1,4]diazepin

4-[4,4-Dimethyl-2-imidazolin-2-yl]-6-(2-chlorphenyl)-11-methyl-2,3,4,5-tetrahydro-8H-[1]benzothieno[3,2-f]-[1,2,4]triazolo[4,3-a][1,4]diazepin

4-[1,4,4-Trimethyl-2-imidazolin-2-yl]-6-(2-chlorphenyl)-11-methyl-2,3,4,5-tetrahydro-8H-[1]benzothieno[3,2-f]-[1,2,4]triazolo[4,3-a][1,4]diazepin

4-Aminocarbonyl-6-(2-chlorphenyl)-11-methyl-2,3,4,5-tetrahydro-8H-[1]benzothieno[3,2-f][1,2,4]triazolo[4,3-a][1,4]diazepin

4-[Morpholin-4-yl-carbonyl]-6-(2-chlorphenyl)-2,3,4,5-tetrahydro-8H-[1]benzothieno[3,2-f]imidazo[1,5-a][1,4]-diazepin

4-[4,4-Dimethyl-2-thiazolin-2-yl]-6-(2-chlorphenyl)-11-methyl-2,3,4,5-tetrahydro-8H-[1]benzothieno[3,2-f]-[1,2,4]triazolo[4,3-a][1,4]diazepin

4-Amino-6-(2-chlorphenyl)-11-methyl-2,3,4,5-tetrahydro-8H-[1]benzothieno[3,2-f][1,2,4]triazolo[4,3-a][1,4]-diazepin

2-(4-Isobutylphenylethyl)-4-(2-chlorpheny)-9-methyl-6H-thieno[3,2-f][1,2,4]triazolo[4,3-a][1,4]diazepin

3-(N-Morpholinyl-methyl)-5-(2-chlorphenyl)-10-methyl-3,4-dihydro-2H,7H-cyclopenta[4,5]thieno[3,2-f]imidazo-[1,2-a][1,4]diazepin

4-(1,2,4-Triazol-1-yl-methyl)-6-(2-chlorphenyl)-11-methyl-2,3,4,5-tetrahydro-8H-[1]benzothieno[3,2-f][1,2,4]-

triazol[4,3-a][1,4]diazepin

4-(1,2,4-Triazol-1-yl-methyl)-6-(2-chlorphenyl)-11-methyl-2,3,4,5-tetrahydro-8H-[1]benzothieno[3,2-f]imidazo-[1,2,-a][1,4]diazepin

Ganz besonders bevorzugt sind

6-(2-Chlorphenyl)-8,9-dihydro-1-methyl-8-[(4-morpholinyl)carbonyl]-4H,7H-cyclopenta[4,5]thieno[3,2-f][1,2,4]-triazolo[4,3-a][1,4]diazepin (Web 2170)

4-(2-Chlorphenyl)-9-methyl-2-[3-(4-morpholinyl)-3-propanon-1-yl]-6H-thieno[3,2-f][1,2,4]triazolo[4,3-a][1,4]-diazepin (Web 2086)

6-(2-Chlorphenyl)-8,9-dihydro-1-methyl-8-[dipropylaminocarbonyl]-4H,7H-cyclopenta[4,5]-thieno[3,2-f][1,2,4]-triazolo[4,3-a][1,4]diazepin (Web 2347)

Die Bezugnahme auf PAF-Antagonisten hierin beinhalten selbstverständlich auch ihre pharmazeutisch verträglichen Additionssalze, ihre Racemate sowie gegebenenfalls ihre optisch aktiven Formen.

Bei einem 52 jährigen männlichen Patienten mit ITP zeigte sich unter der Therapie des PAF-Antagonisten Web 2086 $\widehat{=}$ 4-(2-Chlor- phenyl)-9-methyl-2-[3-(4-morpholinyl)-3-propanon-1-yl]-6H-thieno[3,2-f][1,2,4]triazolo[4,3-a][1,4]diazepin} - in einer Dosis von 4 x 20 mg täglich oral anstelle von Prednisolon -ein so rascher Anstieg der Thrombozyten, wie er bei dem betreffenden Patienten in der seit 3 Jahren bestehenden und diagnostisch gesicherten Erkrankung bei der Behandlung mit Steroiden nie gesehen werden konnte. Nach dem Absetzen nach 10 Tagen trat dann - wie erwartet - ein entsprechender Abfall der Thrombozyten ein (siehe Abbildung 1).

Der Vorteil der Therapie mit PAF-Antagonisten liegt in der soweit bisher beim Menschen bekannt völligen Nebenwirkungsfreiheit gegenüber den bisher in dieser Therapie eingesetzten stark belasteten Therapeutika und einem raschen Anstieg der Thrombozyten. Als Einzeldosis ist ein Dosisbereich zwischen 5 und 50 mg bei oraler Applikation sinnvoll, bei einer intravenösen Applikation sind Einzeldosen zwischen 2,5 und 30 mg empfehlenswert.

In einer weiteren erfindungsgemäßen Ausführungsform kann der PAF-Antgonist in Kombination mit einem Kofaktor in niedrig dosierter Form angewendet werden. Als Kofaktoren kommen immunsuppressiv wirkende Substanzen und auch Steroide in geringer Konzentration in Frage.

Aus dem Stand der Technik ist bekannt, daß immunsuppressive Substanzen - wie z.B. Cyclosporine - zur Behandlung der idiopatischen thrombozytopenischen Purpura vorgeschlagen wurden. Cyclosporine weisen bekanntermaßen starke Nebenwirkungen auf. Die erfindungsgemäße Kombination führt zu einer Verrringerung dieser Nebenwirkungen. Von besonderem Interesse sind in diesem Zusammenhang Arzneimittelkombinationen bestehend aus einem PAF-Antagonisten ausgewählt aus den europäischen Patentanmeldungen 176 928, 268 242, 230 942, 240 899, 194 416, 254 245, 268 242 und 255 028 und einem Cyclosporin - insbesondere Cyclosporin A - Azathioprin oder Prednisolon. Die aufgelisteten europäischen Patentanmeldungen enthalten als zentrales Strukturelement ein Thieno[3,2-f][1,2,4]triazolo[4,3-a][1,4]-diazepin bzw. ein [1,2,4]Triazolo[4,3-a][1,4]benzodiazepin. Von besonderem Interesse sind Mittel, die den PAF-Antagonisten Web 2086, Web 2170 oder Web 2347 und ein Cyclosporin - insbesondere Cyclosporin A - enthalten.

Ein unerwünschter Nebeneffekt von Cyclosporin ist seine Nierentoxizität (11. J. Mikatsch et al. Transplant Proc. 15, 2821-2835 (1983). Die Behandlung von männlichen spontan hypertonen (SH) Ratten mit Clycosporin verursacht eine Schädigung der Nieren, die als Model für die durch Cyclosporin hervorgerufene Nierentoxizität angesehen wird (vgl. hierzu B. Ryffel, H. Siegel, A.M. Müller, R. Hauser, M.J. Nikatsch Transplant Proc. 17, 1430-1431 (1985).

Ein Maß für diese Nierentoxizität ist die Bestimmung des Kreatinin-Serum-Spiegels. Eine Erhöhung des Kreatinin-Serum-Spiegels ist ein Anzeichen für eine Nierenschädigung.

Gruppen von 8 SH Ratten bekamen oral 28 Tage entweder 2, 25 oder 50 mg/kg Cyclosporin A; 2, 25 oder 50 mg/kg Cyclosporin A plus 13 mg/kg Web 2170; 13 mg/kg Web 2170 allein oder die Vehikel (Olivenöl und Wasser allein). Blut wurde vor Versuchsbeginn und 4, 13, 22 und und 29 Tage nach Versuchsbeginn entnommen. Der mittlere Kreatnin-Serum Wert war in den Gruppen der mit Cyclosporin und Web 2170 behandelten Tiere niedriger als in den Gruppen, die Cyclosporin allein bekamen. Dieser Effekt war statitisch signifikant (0,05>p).

Beispielsweise wurden nach 25-tägiger Behandlung folgende mittleren Kreatnin-Serum-Spiegel be-stimmt ($\mu$Mol/ltr).

EP 0 361 077 A2

| Vehikel: | 56 |
|---|---|
| 13 mg/kg WEB 2170 | 55 |
| 2 mg/kg Cyclosporin | 59 |
| 25 mg/kg Cyclosporin | 93 |
| 50 mg/kg Cyclosporin | 71 |
| 13 mg/kg WEB 2170 plus 2 mg/kg Cyclosporin | 53 |
| 13 mg/kg WEB 2170 plus 25 mg/kg Cyclosporin | 65 |
| 13 mg/kg WEB 2170 plus 50 mg/kg Cyclosporin | 67 |

Aufgrund der Ätiologie der ITP (Morbus Werlhof) als Autoimmunerkrankung ist eine Therapie mit PAF-Antagonisten, insbesondere mit 4-(2-Chlorphenyl)-9-methyl-2-[3-(4-morpholinyl)-3-propanon-1-yl]-6H-thieno-[3,2-f][1,2,4]triazolo[4,3-a] [1,4]diazepin auch bei den folgenden Autoimmunerkrankungen, sinnvoll so z.B. bei Autoimmunhaemolytischen Anaemien, autoimmunologisch bedingte Glomerulonephritiden, Thyreoidis Hashimoto, primäres Myxoedem, perniziöse Anaemie, autoimmune atrophische Gastritis, Morbus Addison, iuveniler Diabetes, Goodpasture-Syndrom, idiopathische Leukopenie, primär biliäre Zirrhose, aktive bzw. chronisch aggressive Hepatitis (HBsAg-neg.), Colitis ulcerosa, chronische Polyathritis und systemischer Lupus erythematodes (SLE).

Die Synthese von 4-(2-Chlorphenyl)-9-methyl-2-[3-(4-morpholinyl)-3-propanon-1-yl]-6H-thieno[3,2-f]-[1,2,4]triazolo[4,3-a][1,4]diazepin wie auch die Herstellung galenischer Zubereitungen ist aus der deutschen Offenlegungsschrift DE A1 35 02 392 (Beispiel 1) bekannt. Die Synthesen von Web 2170 und Web 2347 sind aus der europäischen Patentanmeldung 254 245 bekannt. Die Wirkstoffe werden in Form üblicher galenischer Zubereitungen wie z.B. Tabletten, Zäpfchen, Injektionslösungen angewendet. Einzelheiten zu deren Herstellung sind in der europäischen Patentanmeldungen 194 416 und 254 245 offenbart, auf die hiermit inhaltlich Bezug genommen wird.

Arzneimittelkombinationen, die einen PAF-Antagonisten und eine immunsuppressiv wirkend Substanz enthalten, enthalten im allgemeinen bis zu 40 Gew. % der immunsuppressiv wirkenden Substanz, bezogen auf die Gesamtwirkstoffmenge. In Einzelfällen kann der Anteil der immunsuppressiv wirkenden Substanz auch der Hauptbestandteil -d.h. größer 50 Gew %- sein.

Bevorzugt ist ein Anteil zwischen 10 und 20 Gew. %. Der Anteil des PAF-Antagonisten in Kombinationspräparaten beträgt pro Einzeldosis bei oraler Applikation zwischen 5 und 50 mg, bei einer intravenösen Applikation zwischen 2 und 30 mg.

Die erfindungsgemäßen Wirkstoffkombinationen können in Form üblicher galenischer Zubereitungen angewendet werden, so z.B.

## 1. Tabletten

5 Gew.-Teile Wirkstofkombination gemäß der Erfindung
1 Gew.-Teile Stearinsäure
194 Gew.-Teile Traubenzucker
Die Bestandteile werden zu Tabletten von 200 mg verpreßt.

## 2. Ampullenlösungen

5 mg Wirkstoffkombination
45 mg Natriumchlorid
ad 5 ml Aqna pro inj

In Abbildung 1 ist die Anzahl der Thrombozyten in Tausend gegen die Zeit in Tagen aufgetragen. Der Beginn der Therapie erfolgt zum Tag O - erwartungsgemäß -aufgrund einer gewissen Latens - sinkt die Anzahl der Thrombozyten am darauffolgenden Tag noch weiter ab, um im Verlauf der Behandlung auf annähernd Normalwerte von ca. 150 000 sehr rasch anzusteigen. Nach der Beendigung der Therapie am elften Tag sinkt die Anzahl der Thrombozyten ebenfalls erwartungsgemäß wieder ab.

EP 0 361 077 A2

Die Thrombozyten wurden nach bekannten Methoden mit einem Coultex Counter[R] Modell S-Plus II bestimmt.

## Ansprüche

1) Verwendung von PAF-Antagonisten zur Herstellung eines Arzneimittels zur Behandlung von Autoimmunerkrankungen.

2) Verwendung von PAF-Antagonisten zur Herstellung eines Arzneimittels zur Behandlung der ideopatischen thrombocytopenischen Purpura.

3) Verwendung nach Anspruch 1 oder 2, dadurch gekennzeichnet, daß der PAF-Antagonist ein Derivat eines Thieno[3,2-f][1,2,4]triazolo[4,3-a][1,4]diazepins oder eines [1,2,4]-Triazolo[4,3-a][1,4]benzodiazepins ist.

4) Verwendung nach Anspruch 1 oder 2, dadurch gekennzeichnet, daß als PAF-Antagonist Web 2086, Web 2170 oder Web 2347 verwendet wird.

5) Mittel enthaltend einen PAF-Antagonisten und eine immunsuppressiv wirkende Substanz.

6) Mittel nach Anspruch 5, dadurch gekennzeichnet, daß der PAF-Antagonist ein Derivat eines Thieno-[3,2-f][1,2,4]triazolo[4,3-a][1,4]diazepins oder eines [1,2,4]Triazolo[4,3-a][1,4]benzodiazepins ist.

7) Mittel nach Anspruch 5 oder 6 dadurch gekennzeichnet, daß der PAF-Antagonist Web 2086, Web 2170 oder Web 2347 ist.

8) Mittel nach einem der Ansprüche 5 bis 7 dadurch gekennzeichnet, daß die immunsuppressiv wirkende Substanz ein Cyclosporin ist.

9) Mittel nach einem der Ansprüche 5 bis 8 zur Behandlung von Autoimmunerkrankungen.

10) Mittel zur Verminderung der unerwünschten Nebenwirkungen bei der therapeutischen Anwendung von Cyclosporin gekennzeichnet durch einen Gehalt an einer PAF-antagonistischen Verbindung gemäß Anspruch 6 oder 7.

11) Verwendung von PAF-Antagonisten, wie in Anspruch 6 oder 7 definiert, zur Verminderung unerwünschter Nebeneffekte bei der therapeutischen Anwendung von Cyclosporin.

12) Verwendung von PAF-Antagonisten - wie in Anspruch 6 oder 7 definiert - zur Herstellung eines Arzneimittels zur Verminderung unerwünschter Nebeneffekte bei den therapeutischen Anwendung von Cyclosporin.

13) Pharmazeutische Zubereitung enthaltend einen PAF-Antagonisten, eine immunsuppressiv wirkende Substanz und übliche Hilfs- und Trägerstoffe.